# EUROPEAN PATENT SPECIFICATION

(11) **EP 3 288 756 B1**
(45) Date of publication and mention of the grant of the patent: **18.11.2020**
(21) Application number: 15890309.6
(22) Date of filing: 30.04.2015
(51) Int. Cl.: B32B 5/26, B32B 7/04, A61F 13/15, B31F 1/07, D04H 1/593

(54) **IMPROVED EMBOSSED AND APERTURED LAMINATE FOR ABSORBENT ARTICLES AND THE LIKE**
VERBESSERTES GEPRÄGTES UND MIT EINER ÖFFNUNG VERSEHENES LAMINAT FÜR SAUGFÄHIGE ARTIKEL UND DERGLEICHEN
STRATIFIÉ AMÉLIORÉ GAUFRÉ À OUVERTURES POUR ARTICLES ABSORBANTS ET SIMILAIRES

(43) Date of publication of application: 07.03.2018
(73) Proprietor: Kimberly-Clark Worldwide, Inc., Neenah, Wisconsin 54956 (US)
(72) Inventor: HAO, Xueen, Melbourne, Victoria 3165 (AU); PU, Chun Lei, Beijing 100025 (CN); MIAO, Lin, Beijing 100079 (CN); CHEN, Han, Beijing 100176 (CN)
(74) Representative: Dehns
(86) International application number: PCT/CN2015/077988
(87) International publication number: WO 2016/172929

(56) References cited:
- WO-A1-2010/110875
- WO-A1-2012/148935
- WO-A1-2014/155216
- WO-A2-2006/115574
- CN-A- 101 564 913
- CN-A- 102 963 103

## Description

### BACKGROUND OF THE INVENTION

Absorbent articles such as diapers, training pants, incontinence products, feminine hygiene products as well as health care related products such as bandages and other wound dressings have a common goal of rapidly absorbing discharged body fluids such as blood, menses, urine and bowel movements. Typically such products will have a body contacting side and surface which is near or in contact with the wearer's skin, some type of absorbent core and a back sheet or outercover that will prevent the retained fluids from exiting the product and possibly soiling the surrounding areas including the wearer's clothing.

Thus, it is desirable for such products to rapidly take in fluids, pass them to subjacent layers in the product and provide air circulation adjacent the wearer's skin to promote skin wellness. Air circulation allows drying of the skin to prevent skin irritation such as diaper rash in the case of diapers, training pants and incontinence devices. Air circulation also provides increased comfort by allowing the body contacting material, often referred to as a top sheet or liner, to dry out. In addition, comfort and dryness can be further enhanced by minimizing the amount of the liner material that is in direct contact with the skin. This also facilitates a reduction in what is called "rewet" which is the backflow of fluid from the absorbent core onto the liner. As these are desirable attributes for such products, a number of materials and products have attempted to provide these results.

Dryness, softness and breathability are key attributes in personal care absorbent articles. Currently most body side liner materials are very planar and two-dimensional, even when laminates and other multi-layer structures are employed. While aperturing and embossing are employed with such structures, there is still a need for laminates which provide rapid fluid intake, minimal body contact and reduced rewet. In addition, it is desirable for such materials, even if more three-dimensional in design, to be able to maintain a more three-dimensional nature even after subjected to compressive forces.

While the foregoing are examples of attempts to provide materials with the desired fluid handling properties, there is still a need for improved materials in this regard. The present invention is directed to a laminate which can be used in this regard in conjunction with personal care absorbent articles including, but not limited to, diapers, training pants, incontinence garments, feminine hygiene products such as sanitary napkins and panty liners as well as other absorbent products including bandages, wound dressings and various types of wiping products.

Related art includes WO2012/148935A1 which discloses a method for forming a web, and WO2014/115216A1 which discloses coordinated apertured and embossed topsheet layer materials, and absorbent articles containing such.

### SUMMARY

In one aspect, the present invention provides a process for forming a laminate including a fibrous nonwoven web first layer and a second layer as claimed in claim 1.

The first roll surface of the first roll and the second roll surface of the second roll are positioned in mating relationship to form a nip between the first roll surface of the first roll and the second roll surface of the second roll with the first plurality of embossing pins and the first plurality of aperturing pins defining a first roll embossing region in the nip between the first roll and the second roll. The first roll further defines a first roll merging region, a first roll bonding region and a first roll take-off region. A bonding apparatus is positioned adjacent the first roll surface of the first roll in the first roll bonding region and each of the first roll and the second roll are rotated in a counter-rotating relationship with respect to one another.

To utilize the process there is provided a first layer and a second layer. The first layer is formed of a fibrous nonwoven web having a first layer top surface and a first layer bottom surface separated by a first layer thickness. The second layer has a second layer upper surface and a second layer lower surface separated by a second layer thickness.

In use, the first layer top surface of the first layer is fed into the nip adjacent the first roll surface of the first roll to simultaneously form a first plurality of embossments and a first plurality of apertures in the first layer in the first roll embossing region of the first roll. The second layer upper surface of the second layer is then fed onto the first layer bottom surface of the first layer in the first roll merging region of the first roll with the second layer upper surface of the second layer contacting the first plurality of embossments in the first layer.

In the next portion of the process, a bonding apparatus located in the first roll bonding region is used to bond the second layer upper surface of the second layer to the first plurality of embossments of the first layer in the first roll bonding region of the first roll to form the laminate with an air gap located between the first layer bottom surface of the first layer and the second layer upper surface of the second layer. Once the laminate is formed, it is removed from the first roll in the first roll take-off region of the first roll.

If desired, the process can further include a first plurality of embossing recesses located on the other one of the first roll and the second roll that does not have the first plurality of embossing pins such that the first plurality of embossing pins can mate with the first plurality of embossing recesses located in the other roll.

If desired, in addition, the process can further include a first plurality of aperturing recesses located on the other one of the first roll and the second roll that does not have the first plurality of aperturing pins such that the first plurality of aperturing pins can mate with the first plurality of aperturing recesses located in the other roll. Still further, the embossing pins and recesses as well as the aperturing pins and recesses can be apportioned between the two rolls.

If desired, the process can further include the step of aperturing the second layer prior to feeding the second layer into the first roll merging region of the first roll.

If desired, the bonding step of the process can include ultrasonically bonding the first layer to the second layer or it can use heat and pressure to bond the first layer to the second layer.

If desired the bonding step can cause apertures to be formed in an area where the second layer upper surface of the second layer is bonded to at least a portion of the first plurality of embossments in the first layer.

According to another aspect, the present invention provides a laminate comprising a first fibrous nonwoven layer and a second layer, as claimed in claim 6.

If desired, the second layer of the laminate can define a plurality of spaced-apart second layer apertures therein at least a portion of which extend from the second layer upper surface of the second layer to the second layer lower surface of the second layer to create liquid passageways therebetween. Additionally, if desired, at least a portion of the plurality of first layer embossment side walls can define apertures therein.

In some embodiments, the second layer of the laminate can be a fibrous nonwoven web or, alternatively, an apertured film.

Applications of the present invention include an absorbent article including a liquid permeable top sheet and a liquid impermeable outer cover with an absorbent core positioned therebetween. The laminate can form any portion of the absorbent article including a portion of the absorbent article including the top sheet. If desired, the absorbent article can further include a liquid acquisition layer positioned between the topsheet and the absorbent core and the top sheet can be bonded to the liquid acquisition layer.

In specific embodiments the absorbent article can be, for example, a diaper, training pant or an adult incontinence product such as a pull-on pant or diaper. It can also be a feminine hygiene product such as a sanitary napkin or a panty liner.

### BRIEF DESCRIPTION OF THE DRAWINGS

Figure 1A is a cross-sectional side view of a laminate according to the present invention.
Figure 1B is a cross-sectional side view of an alternate laminate according to the present invention.
Figure 1C is a cross-sectional side view of a portion of an alternate laminate according to the present invention.
Figure 2 is top plan view of the first layer top surface of a laminate such as in Figures 1A, 1B and 1C showing the embossments and apertures according to the present invention.
Figure 2A is top plan view of the first layer top surface of a laminate according to the present invention using dashed lines to show a first array of embossments and a first array of apertures.
Figure 3 is a side view of a schematic of a process for forming a laminate according to the present invention.
Figure 4 is an enlarged side view of the nip area of the first roll and second roll of the process shown in Figure 3.
Figure 5 is an enlarged view of the nip area and first roll and second roll of the process for forming a laminate according to the present invention.
Figure 6 is a partial cut-away, top plan view of an absorbent article, in this case a sanitary napkin, employing the laminate of the present invention.
Figure 7 is a partial cut-away, top plan view of an absorbent article, in this case a diaper, employing the laminate of the present invention.

### DETAILED DESCRIPTION

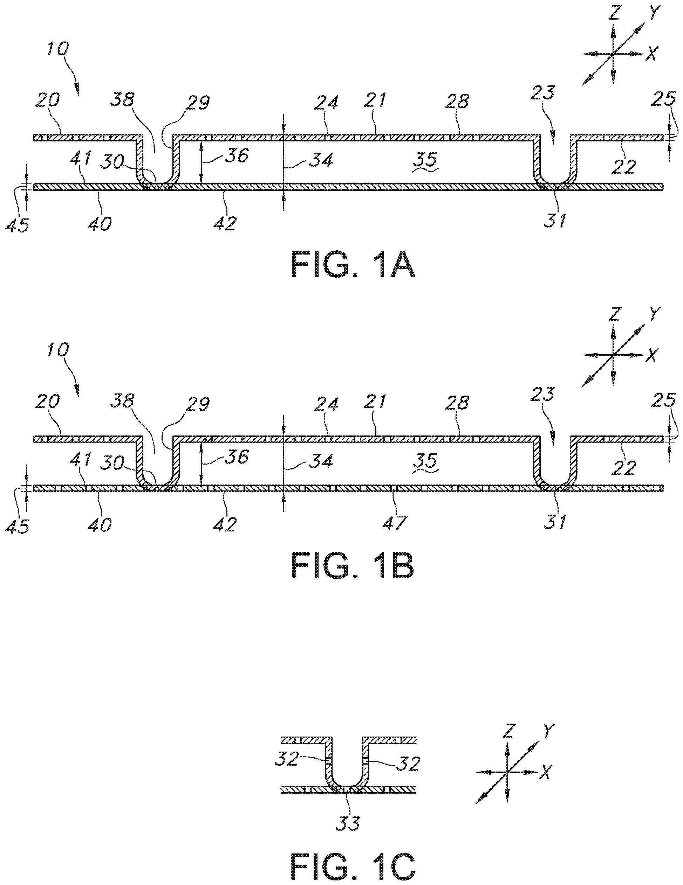

Turning to Figure 1A, there is shown a laminate 10 including a first layer 20 and a second layer 40. For references purposes, the laminate 10 can be regarded as residing primarily in an X-Y plane with the thickness of the laminate extending in the Z or vertical direction. The first layer 20 is a fibrous nonwoven web having a first layer top surface 21 and a first layer bottom surface 22 which define a first layer thickness 25 therebetween. As will be explained in further detail below with respect to the process for forming the laminate 10, the first layer 20 is attached to a second layer 40 which has a second layer upper surface 41 and a second surface lower surface 42 which define a second layer thickness 45 therebetween.

To separate the first layer 20 from the second layer 40, the first layer 20 is provided with a plurality of downwardly depending (in the above-referenced Z or vertical direction) embossments 23. By "downwardly depending" it is meant that material from the X-Y plane of the first layer 20 is permanently displaced in the vertical or Z-direction so as to form depressions or wells 38 which extend from the first layer bottom surface 22 towards and contacting the second layer upper surface 41. As shown in Figures 1A and 1B, the first layer embossments 23 form depressions or wells 38 which have embossment sidewalls 29 and embossment bottoms 30 which are bonded to the second layer upper surface 41 by way of embossment bond points 31. As will be explained in further detail below with respect to the laminate formation process, the embossment bond points 31 are preferably created by fusion of the fibers of the first layer 20 with and to the fibers or material (as when the second layer 40 is a film or film/nonwoven laminate) forming the second layer 40. Alternatively, however, this bonding of the two layers (20 and 40) may be achieved by other means such as adhesives and other means or a combination of such bonding techniques.

When the laminate 10 is being used as a body side layer also referred to as a liner material or top sheet in personal care absorbent articles, rapid fluid intake and transfer of fluids down into the lower, internal layers of the absorbent product is a highly desirable attribute. Once the fluids have been transferred to the internal portions of the absorbent product, it is also desirable that the fluids so delivered not flow back up to the top surface of the laminate/top sheet of the product. This is referred to as rewet. To facilitate such properties, first layer 20 of the laminate 10 is provided with a plurality of first layer apertures 24 which extend from the first layer top surface 21 down and through the first layer bottom surface 22. See Figures 1A and 1B. As shown in Figures 1A and 1B, these first layer apertures 24 are somewhat two-dimensional as there is no material from the aperturing process shown depending downwardly in the Z-direction below the first layer bottom surface 22 towards the second layer upper surface 41. However, in some instances it is possible during the aperturing process for fibers from the first layer 20 to be displaced (not shown) downwardly to form funnel-like shapes (also not shown) which can increase the capillary action of the apertures 24 to draw body fluids down into the laminate 10 and subjacent layers and further retard rewet - the flow of absorbed fluids back towards to first layer top surface 21.

As a result of the use of the first layer embossments 23, an air gap 35 is created between the first layer 20 and the second layer 40 and in particular, between the first layer bottom surface 22 and the second layer upper surface 41. These two surfaces (22 and 41) define an air gap thickness 36. The air gap thickness 36 can range between about 0.1 and about 15 millimeters (mm), alternatively, between about 0.1 and about 4 mm and, alternatively, between about 0.1 and about 1 mm.

The first layer 20 can be made from a wide variety of fibrous nonwoven webs such as through air bonded carded webs (TABCW), thermally bonded calendered webs, airlaid webs, spunbond webs, spunlace webs, meltblown webs, and apertured polymeric films such as polyethylene films. Through air bonded carded webs are particularly useful as the first layer 20 due to their softness and hand. With respect to the fibrous webs used for the first layer, the fibers (also referred to as filaments) can be more continuous such as are encountered with spunbond and meltblown fibrous webs. Alternatively the fibrous nonwoven webs can be made from staple fibers. Both the filaments and fibers can include, but are not limited to, single component fibers and multi-constituent fibers such bicomponent fibers. Suitable fiber compositions include, but are not limited to, polyolefins such as polypropylene and polyethylene as well as polyester, viscose, rayon and cotton. Due to their combinations of bonding capability, softness and strength, bicomponent fibers with polyethylene sheaths and polypropylene or polyester cores have been found to work particularly well with the laminate 10 described herein. Fiber deniers for the fibers can be between about 0.05 and about 5 denier, alternatively between about 1.2 and about 3 denier and, alternatively between about 1.5 and about 2.5 denier.

The second layer 40 can utilize the same materials as the first layer 20 including fibrous nonwoven webs and film layers. Because the second layer will not typically come in contact with the end-user, it may be made with higher denier fibers that the first layer 20. The fibers of the second layer 40 can be within the same ranges as denoted for the first layer 20 but can also be between about 0.05 and about 12 denier, alternatively between about 1 and about 9 denier and, alternatively between about 2 and about 6 denier.

If desired, either one or both of the first layer 20 and the second layer 40 may be treated with fluid-handling treatments such as surfactants to increase fluid flow down into the laminate 10 and the subjacent layers when the laminate 10 is included in end-use products such as the aforementioned absorbent articles.

The first layer 20 will typically have a thickness of between about 0.1 and about 5.0 millimeters (mm), alternatively between about 0.3 and about 1.5 mm and alternatively between about 0.4 and about 0.6 mm. Still further ranges and specific thicknesses may be used depending upon the particular end use of the laminate 10. The basis weight of the first layer 20 will also depend upon the particular end use but will typically have a basis weight of between about 5 and about 100 grams per square meter (gsm), alternatively between about 12 and about 50 gsm and alternatively between about 20 and about 50 gsm. Still further ranges and specific basis weights may be used depending upon the particular end use of the laminate 10.

The second layer 40 can have a basis weight of between about 10 and about 150 gsm, alternatively between about 15 and about 60 gsm, and, alternatively between about 20 and about 35 gsm. The second layer thickness 45 of the second layer 40 can be between about 0.2 and about 10 mm, alternatively between about 0.3 and about 2 mm, and alternatively between about 0.4 and about 1 mm. The overall thickness 34 of the laminate 10 can be between about 0.5 and about 20 mm, alternatively between about 1 and about 5 mm, and alternatively between about 1.5 and about 3 mm. The laminate 10 can have a total basis weight of between about 15 and 250 grams per square meter (gsm), alternatively between about 27 and about 110 gsm and, alternatively between about 40 and about 60 gsm.

The size, shape and number of first layer embossments 23 can be varied depending upon the particular end attributes of the overall laminate 10. Larger numbers of embossments will provide greater structural rigidity between the first layer 20 and the second layer 40. The number and size of the embossments can also depend on the size, shape and density of the fibers as well as the degree of fiber-to-fiber bonding between individual fibers in the first layer 20 and the second layer 40. Larger fibers with more fiber-to-fiber bonding will create more rigid embossments as will the degree of fusion during the bonding of the embossments bottoms 30 to the second layer upper surface 41. Also, depending upon the degree of fusion of the fibers that takes place in the embossments 23, the sidewalls 29 and bottoms 30 of the first layer embossments 23 may melt to a point that further stiffening and rigidity can be built into the embossments 23. Additionally, it is possible to subject the laminate 10 to post treatments such as additional heating steps to further set and fuse the fibers forming the first layer embossments 23.

The size of the embossments 23 can be varied depending upon the end use. Also, the shape of the embossments 23 can be varied and can include, but is not limited to, circles, ovals, squares, rectangles and other multi-sides openings such as diamonds, triangles, octagons, hexagons and other polygon shapes. The shapes may also be regular or irregular. Still further, combinations of embossment shapes and sizes can be used if so desired. They also may have aspect ratios (major to minor axes ratios) of between about 1:1 to about 20:1, alternatively between about 1:1 to about 5:1, and still further between about 1:1 to about 3:1.

The first layer embossments 23 can have a depth (as measured from the first layer top surface 21 to the embossment bottom 30) of between about 0.2 and about 20 mm, alternatively between about 0.4 and about 5.5 mm and, alternatively between about 0.5 and about 1.6 mm. The first layer embossments 23 can have an individual embossment area (as measured at the first layer top surface 21) of between about 1 and about 35 square millimeters (mm²), alternatively between about 1.5 and about 20 mm² and, alternatively between about 2.5 and about 8 mm². The overall open area of the plurality of embossments per unit area of the first layer 20 as measured at the first layer top surface 21 can be between about 5 and about 55 percent, alternatively between about 10 and about 35 percent and, alternatively between about 15 and about 25 percent.

The first layer apertures 24 can have a diameter (as measured as the greatest distance between two sides of the aperture without touching an intermediate side edge) of between about 0.2 and about 10 mm, alternatively between about 0.2 and about 5 mm and, alternatively between about 0.5 and about 2 mm. The first layer aperture spacing (as measured as the distance between the two closest respective aperture edges of two adjacent apertures at the first layer top surface 21) can be between 0.5 and about 20 mm, alternatively between about 0.5 and about 15 mm and, alternatively between about 1 and about 6 mm. The overall open area of the plurality of apertures per unit area of the first layer 20 as measured at the first layer top surface 21 can be between about 1 and about 40 percent, alternatively between about 5 and about 30 percent and, alternatively between about 10 and about 20 percent.

The size, shape and number of first layer aperture 24 can be varied depending upon the particular end attributes of the overall laminate 10. Also, the size, shape and number of first layer apertures 24 will affect the overall fluid transfer properties of the first layer 20 and the resultant laminate 10. The first layer apertures 24 can have various shapes and combinations of shapes including, the same or different shapes as described above with respect to the first layer embossments 23. Also, combinations of sizes of apertures can be used. For example, if the laminate 10 is being used as a top sheet for a personal care absorbent article such as a diaper of sanitary napkin, a middle region of the topsheet may use larger aperture sizes that the apertures located in the lateral portions of the top sheet or in the peripheral portions of the top sheet.

When the laminate 10 is being used as a body side layer of a personal care product such as a diaper, diaper pant, training pant, feminine hygiene product, adult diaper, incontinence product, etc., it is desirable that the body contacting layer be soft to the touch. This is why it is preferable that the first layer 20 be a fibrous nonwoven web. The second layer 40 will typically not come in contact with the body of the user, thus, while it is still desirable that the second layer 40 also be a fibrous nonwoven web, it may be formed from other materials such as films and film/nonwoven laminates. In such situations, it is necessary that the second layer 40 and the laminate 10 as a whole be able to pass body fluids including liquids such as urine, menses and blood and to some extent solids such as feces. As a result, in such situations, it will be advantageous for the film to have apertures, slits or some other type of fluid passageways. When the second layer 40 comprises a fibrous nonwoven web, it may use the same types, thicknesses and basis weights of fibrous nonwoven webs as delineated above with respect to the first layer 20. Thus, the first layer 20 and the second layer 40 may be the exact same material or different materials. For example, the first layer 20 may be a through-air bonded carded web and the second layer 40 may be a spunbond nonwoven web or an airlaid web.

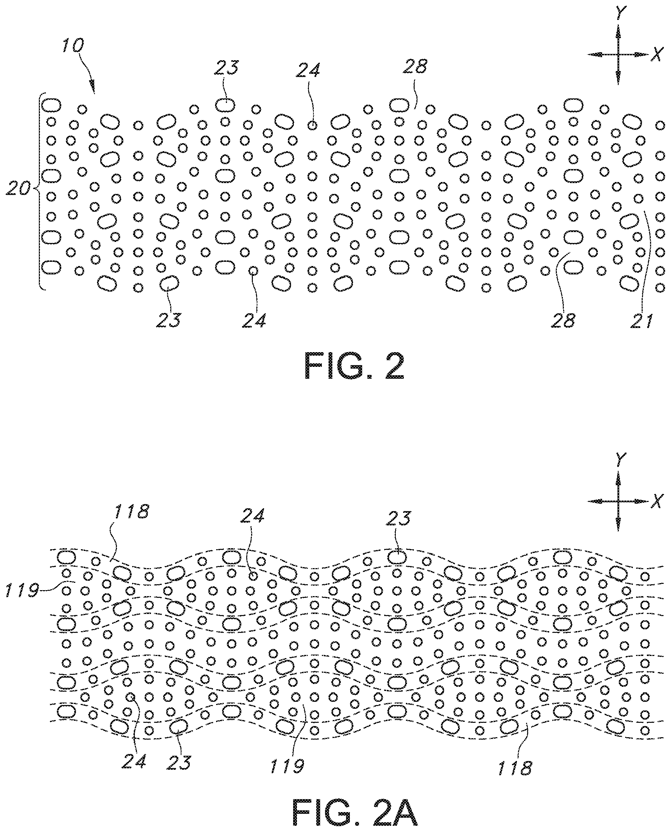

The area of the first layer top surface 21 not taken up by the first layer embossments 23 and the first layer apertures 24 is referred to as the first layer land area 28. See figures 1A, 1B and 2. Generally, when the laminate 10 is being used as a top sheet in a personal care absorbent article, it is desirable to minimize the land area 28 as this also reduces skin contact area which if damp, can lead to skin irritation on the user. The land area 28 can range between about 5 percent and about 95 percent, alternatively between about 35 percent and about 85 percent, and still further between about 55 percent and about 75 percent per unit area of the first layer top surface 21. The percent land area per a unit area of the first layer top surface 21 is the surface area of the land area 28 located in the defined unit area (discounting the area of the embossments 23 and the apertures 24 per the same unit area) divided by the total area of the unit area being measured with the quotient multiplied by 100.

The laminate 10 shown in Figure 1A has a first layer 20 with a first layer thickness 25 of 0.6 mm. The second layer 40 has a second layer thickness 45 of 0.4 mm. The air gap 35 between the first layer bottom surface 22 of the first layer 20 and the second layer upper surface 42 of the second layer 40 has an air gap thickness 36 of 0.8 mm and the overall thickness 34 of the laminate 10 of 1.8 mm.

Turning to Figure 1B, a second laminate 10 is shown. In this embodiment, the laminate 10 is shown with the second layer 40 having optional second layer apertures 47. Such additional aperturing will further increase fluid flow from the first layer top surface 21 down into the laminate 10 and any adjacent sublayers in the event the laminate 10 is incorporated into another structure such as the above-mentioned personal care absorbent article. The second layer apertures 47 can have various shapes and combinations of shapes including, the same or different shapes as described above with respect to the embossments 23 and the apertures 24 in the first layer 20. The size of the apertures can range between about 0.1 and about 10 mm, alternatively between about 0.2 and about 5 mm, and still further between about 0.5 and about 2 mm with the size being measured across the major axis of the aperture in the second layer 40. Also, combinations of sizes of apertures can be used. For example, if the laminate 10 is being used as a top sheet of a personal care absorbent article such as a diaper of sanitary napkin, a middle region of the second layer 40 may use larger aperture sizes that the apertures located in the lateral portions of the second layer 40 or in the peripheral portions of the second layer 40 being used as a part of the top sheet. Alternatively, if the first layer 20 has larger apertures 24 in the central portion of the top sheet (the portion of the top sheet acting as the target zone for the deposited body fluids), then the lateral or peripheral portions of the second layer 40 may have the larger size apertures 47 to allow for greater distribution of the absorbed body fluids down into the underlying absorbent core and other layers outside the target zone of the top sheet. These second layer apertures 47 may be formed in the same manner as the first layer apertures 24.

Referring again to Figure 1B, if desired, the first layer embossments 23 may have embossment side wall apertures 32. Further, the first layer embossments 23 may be provided with embossment bottom apertures 33. The size of the embossment side wall apertures 32 and the embossment bottom apertures 33 may be in the same size range as the apertures 24 in the first layer and the apertures 47 in the second layer 40. Still further, it should be noted that the second layer apertures 47, embossment side wall apertures 32 and embossment bottom apertures 33 may be used alone or in combination with one another.

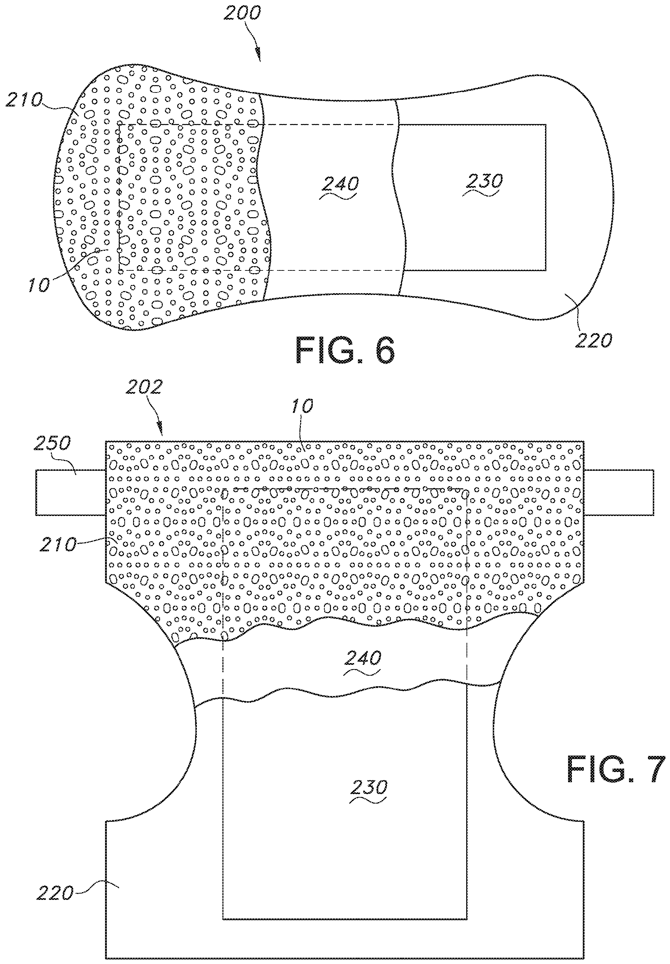

The overall design of the embossments 23 and apertures 24 can be varied to meet the needs of the particular laminate and/or product into which the laminate 10 will be incorporated. Turning to Figure 2A it can be seen that the embossments 23 form a first pattern or array of embossments 118 and the apertures 24 form a second pattern or array of apertures 119. To further facilitate viewing of these arrays 118, 119, a series of dashed lines are shown in Figure 2A to further distinguish the arrays. These dashed lines are for illustration purposes only. The first array 118 and the second array 119 run longitudinally in the X direction and alternate with one another in the "Y" direction. Each of the arrays has a wavy or sinusoidal pattern in the X direction. This has been found to be desirable to break up fluid flow, especially when the laminate 10 is being used, for example, as a top sheet 210 for a personal care absorbent article such as the sanitary napkin 200 in Figure 6 or the diaper 202 in Figure 7 of the drawings. As shown in these figures, (Figures 6 and 7), the longitudinal axis of the arrays 118 and 119 are aligned with the longitudinal axes of the products 200 and 202. Alternatively, the arrays 118 and 119 may be run in the transverse direction or at an angle to the longitudinal and transverse axes of the product.

Other designs as to the first and second arrays 118 and 119 are also within the scope of the present invention. Straight lines, circles, ovals and fanciful designs may be used for either or both of the arrays. In addition, one array may partially or wholly encircle the other array. Further, additional arrays, beyond a first and second arrays, may be used in the design of a laminate 10 according to the present invention.

### Process:

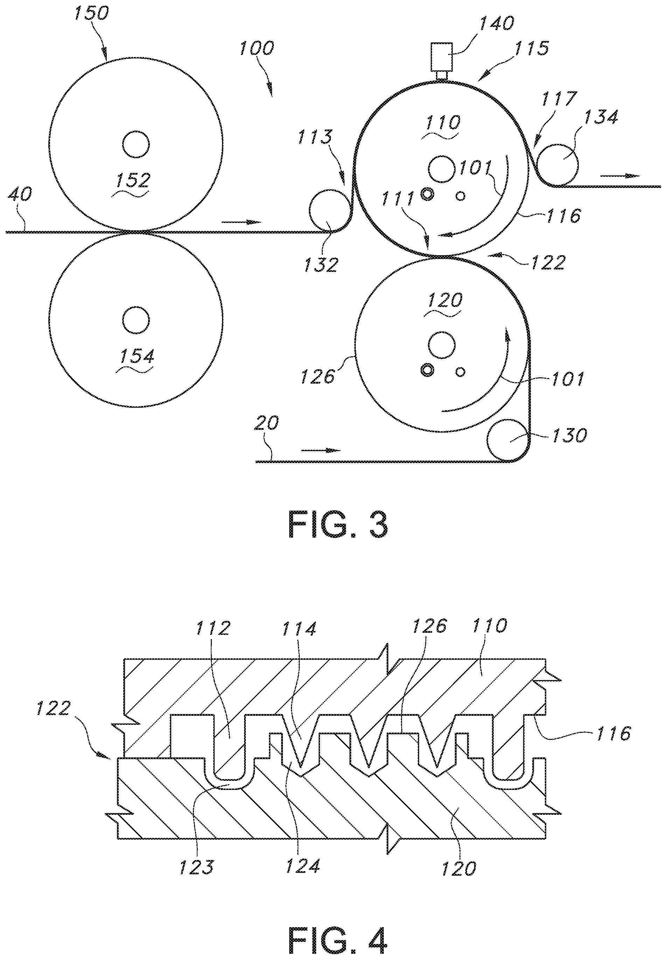

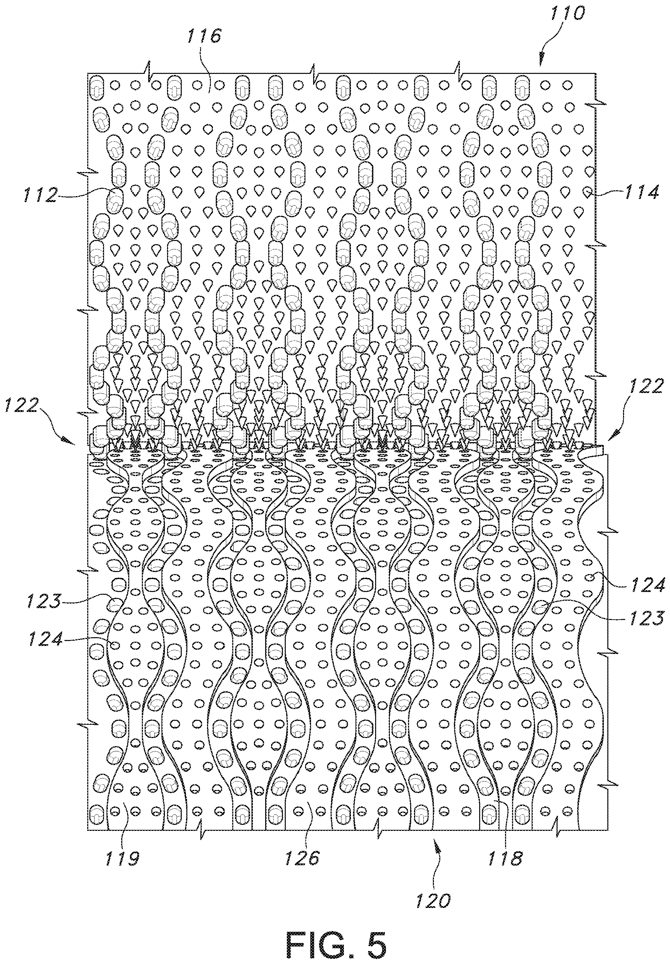

A process 100 for making the laminate 10 is shown in Figures 3, 4 and 5 of the drawings. Referring to Figure 3, the process 100 includes a counter-rotating first roll 110 and second roll 120 with the direction of counter rotation shown by arrows 101. The first roll 110 and the second roll 120 define a nip 122 therebetween formed by the first roll land area 116 and the second roll land area 126. Extending outwardly from the first roll land area 116 of the first roll 110 are a plurality of embossing male pins 112 and aperturing male pins 114 set out in individual patterns. An enlarged view of the first roll 110 and the second roll 120 is shown in Figure 5 with the design of embossing pins and aperturing pins configured to create the embossing and aperturing pattern shown in Figure 2 of the drawings.

The embossing male pins 112 are shaped to form the first layer embossments 23 shown in Figures 1A, 1B, 1C and 2, and the aperturing male pins 114 are shaped to form the first layer apertures 24. Generally the embossing pins 112 will be longer that the aperturing pins 114 and the length of the embossing pins 112 will dictate the air gap 35 between the first layer 20 and the second layer 40. A particular advantage of the equipment and process of the present design is that it allows for the simultaneous aperturing and embossing of the first layer 20 thereby increasing the efficiency and speed of the present process. In addition, by having the aperturing and embossing pins in the same location of the same process, problems with the indexing of the first layer embossments 23 and first layer apertures 24 is minimized.

The second roll 120 has a plurality of embossing female recesses 123 and aperturing female recesses 124 which are in respective mating relationship with the embossing male pins 112 and aperturing male pins 114 in first roll 110 so that the respective pins mate with the respective recesses as the first roll 110 and the second roll 120 are counter-rotated in the direction of arrows 101. The size of the recesses (123 and 124) should be designed to be able to receive the respective pins (112 and 114) and the material from the first layer 20. To facilitate the aperturing and embossing, the first roll 110 and/or the second roll 120 can be heated and/or cooled using conventional means or they may be run at ambient conditions.

In the specific process shown in Figures 3 through 5, the nip 122 between the first roll surface or land area 116 and the second roll surface or land area 126 is 0.8 mm with the first roll embossing pins 112 having a height (as measured from the first roll land area or surface 116 to the end of the embossing pin 112 of 2.4 mm. The first roll aperturing pins have a height (as measured from the first roll surface 116 to the end of the aperturing pin 114 of 2.0 mm.

In alternate designs, not shown, the second roll 120 may have a malleable surface instead of recesses that will deform when contacted by the embossing male pins 112 and aperturing male pins 114 of the first roll 110. In yet further alternate designs, some portion of the pins and recesses may be relocated from the first roll 110 to the second roll 120 and vice versa.

To increase the efficiency and speed of the present process 100, the first roll 110 defines a first roll embossing region 111, a first roll merging region 113, a first roll bonding region 115 and a first roll take-off region 117. The nip 122 is located in the embossing region 111 and a bonding apparatus 140 is located in the first roll bonding region 115.

In operation, the first layer material 20 is fed onto the second roll 120 and into the nip 122 in the first roll embossing region 111. As shown in Figure 3, an optional guide roll 130 may be located adjacent the second roll 120 at a location that allows sufficient contact and dwell time of the first layer material 20 on the second roll surface 126 of the second roll 120 to pre-heat the first layer material 20 in the case where the second roll 120 is heated. As the first layer material 20 travels around the second roll 120, it enters the nip 122 where it is apertured and embossed by the intermeshing aperturing and embossing pins and recesses of the first and second rolls (110 and 120). As the first layer material 20 exits the nip 122, the first layer top surface 21 stays in contact with the first roll surface 116 of the first roll 110 and the first layer bottom surface 22 is disposed away from the first roll 110 and supported by the first layer embossing pins 112.

In the first roll merging region 113, the second layer 40 is fed onto the exposed first layer bottom surface 22. To facilitate the deposition of the second layer 40 into the first roll merging region 113, an optional guide roll 132 may be employed to maintain sufficient tension to retain the second layer upper surface 41 in contact with the first layer bottom surface 22.

As the now juxtaposed first and second layers 20 and 40 continue to travel around the first roll 110, they move into the first roll bonding region 115 where bonding of the two layers (20 and 40) takes place to form the laminate 10. As previously mentioned, the first roll 110 may be heated to facilitate the bonding step in the bonding region 115. Adjacent the first roll surface 116 of the first roll 110 in the bonding region 115 there is located a bonding apparatus 140. As shown in Figure 3, the bonding apparatus 140 is an ultrasonic bonding unit. Such ultrasonic bonding equipment is well known to those in the art of laminate bonding. As the first and second layers (20 and 40) pass between the surface 116 of the first roll 110 and the ultrasonic bonding equipment 140, sufficient energy is imparted to the first and second layers (20 and 40) to fuse the second layer upper surface 41 to the embossment bottoms 30 formed in the first layer bottom surface 22 thereby forming the embossment bond points 31 and the resultant laminate 10 shown in Figures 1A, 1B and 1C.

As the now-formed laminate 10 leaves the first roll bonding region 115, it travels to the first roll take-off region 117 where the laminate 10 is removed for further processing or wind-up on a take-up roll (not shown). As with the other regions on the first roll 110, an optional guide roll 134 may be employed.

In an alternate embodiment, not shown, the ultrasonic bonding apparatus 140 may be replaced by a bonding roll (not shown) which supplies sufficient pressure against the second layer lower surface 42 of the second layer 40 to cause the bonding of the two layers (20 and 40) to one another. Further, if desired, the alternate bonding roll may be heated to facilitate the bonding process.

The laminate 10 shown in Figures 1B and 1C has a second layer 40 with second layer apertures 47. In this regard, if desired, the second layer material 40 may be pre-apertured prior to entering the process 100 shown in Figure 3. Alternatively, a further process step may be added to the process shown in Figure 3 to create the optional second layer apertures 47 shown in Figures 1B and 1C. Specifically, an optional second layer aperturing apparatus 150 may be added to the process 100. The second layer aperturing apparatus 150 may include an aperturing roll 152 and an anvil roll 154. While the rolls 152 and 154 may be run at ambient temperatures, if desired, either or both of the rolls 152 and 154 may be heated and/or cooled to facilitate the aperturing process.

An enlarged view of the first roll 110 and the second roll 120 is shown in Figure 4 with the design of embossing pins and aperturing pins configured to create the embossing and aperturing pattern shown in Figures 2 and 2A of the drawings.

### Product Applications:

The laminate 10 can be used in a wide variety of applications including, but not limited to, absorbent articles and in particular, personal care absorbent articles designed to be worn against or around the body to absorb body exudates. Examples of such articles include, but are not limited to, diapers, diaper pants, training pants also known as pull-on diapers or pants, adult incontinence products and feminine care products such as sanitary napkins, panty liners, etc. Turning to Figures 6 and 7 of the drawings there are shown two exemplary absorbent articles 200 and 202. In Figure 6 the absorbent article is a sanitary napkin 200 and in Figure 7 the absorbent article is a diaper 202. In both figures, like reference numerals are used for like elements. In both instances the laminate 10 is used as the liquid pervious top sheet 210. The respective articles 200 and 202 also include a liquid impervious back sheet or outercover 220 which is typically joined to the top sheet 210 either directly or indirectly (typically around the periphery of the product) and an absorbent core 230 disposed between the top sheet 210 and the back sheet or outercover 220. The bottom surface of the top sheet 210 may be bonded to the absorbent core 230 either directly or indirectly through intermediate layers. Optionally, the articles 200 and 202 may include other layers such as what is termed a surge layer, transfer layer or spacer layer 240 located between the absorbent core 230 and the top sheet 210. In such situations, the surge layer 240 is typically bonded either directly or indirectly to the topsheet 210 and additionally or alternatively to the absorbent core 230. While the laminate 10 is shown as being used for the topsheet 210, it may be used for any of the other layers including, but not limited to, the surge layer 240 and the absorbent core 230. When the article is a diaper 202 such as is shown in Figure 7, the article may further include fastening means 250 such as mechanical hook and loop fasteners. In alternate versions, not shown, the diaper may be of a closed or pull-on design where the side edges are sealed to one another.

In yet other applications, the laminate 10 may be used as a wet or dry wipe as in the case of personal care wipes for babies, children and adults. Such wipes may also be employed as cleaning wipes for household and other uses. In such applications, the laminate may be loaded with cleaning and other compounds. Further, it may be treated or impregnated with other materials such as shoe polish, medications, facial creams, lotions, etc. In this regard, the air gap 35 may be used as an area to store and subsequently deliver such additional materials. The laminate 10 may also be used for medical and health care applications.

### Thickness Test:

The thickness value of a selected sample is determined using a thickness tester which includes a granite base having a vertical clamp shaft extending from the top surface of the granite base which is flat and smooth. A suitable granite base is a Starret Granite Base, model 653G (available from The L.S. Starrett Company, having a place of business located in Athol, Mass., U.S.A.) or equivalent. A clamp arm is secured to the clamp shaft at one end of the clamp arm, and a digital indicator is secured to the clamp arm at the opposing end. A suitable indicator is a Mitutoyo ID-H Series 543 Digimatic Indicator (available from Mitutoyo America Corp., having a place of business located in Aurora, III., U.S.A.) or equivalent. Extending downward from the indicator is a vertically-movable plunger.
To perform the procedure, a block having a length of 130mm, a width of 45 mm and a thickness of 17mm and a weight of 120 grams is placed onto the granite base. The block is constructed of acrylic and is flat and smooth on at least the bottom surface. The thickness and weight of the block is configured such that the thickness tester provides a pressure to the sample of 0.02kPa (0.029 psi). Next, the thickness tester is gently lowered such that the bottom surface of the plunger is in direct contact with the top surface of the block at the longitudinal and transverse center of the block, and the plunger length is shortened by about 50%. The digital indicator is then tared (or zeroed) by pressing the "zero" button. The digital display of the digital indicator should display "0.00 mm" or equivalent. The thickness tester is then raised and the block is removed. The test sample is then placed onto the top surface of the granite base and the block is gently placed on top of the test sample such that the block is substantially centered longitudinally and transversely on the sample. The thickness tester is then gently lowered again onto the block such that the bottom surface of the plunger is in direct contact with the top surface of the block at the longitudinal and transverse center of the block, and the plunger length is shortened by about 50%, to provide a pressure of 0.02kPa (0.029 psi). After 3 seconds, the measurement from the digital display is recorded to the nearest 0.01 mm.

### Examples:

One advantage of the present invention is the ability of the laminate 10 to create a more three-dimensional structure due to the separation between the first layer 20 and the second layer 40 and the air gap 35 therebetween. An important aspect of the laminate 10 in this regard is to maintain this three-dimensional aspect even after being subjected to compressive loads. For example, when the laminate 10 is used as a top sheet 210 in an absorbent article such as is shown in Figures 6 and 7, the three-dimensionality should be able to sustain compressive forces.

To demonstrate the compression resilience of the laminate 10 as compared to non-apertured, non-embossed layers of materials, a series of samples were prepared and tested. The first layer material was a through air bonded carded web (TABCW) made entirely from 38 millimeter (mm), two denier polyethylene sheath/polyester core bicomponent staple fibers. The first layer had a basis weight of 24 grams per square meter (gsm). The second layer material was also a TABCW made from 38 mm, three denier polyethylene sheath/polyester core bicomponent staple fibers. The second layer had a basis weight of 30 gsm and was not apertured.

Samples designated "A" were made in a non-laminated configuration by layering together layers of the above-described first material (24 gsm) with the second material (30 gsm). In samples where multiple layers were used, the first and second materials were layered in an alternating fashion. Neither layer (24 gsm and 30 gsm) of the non-laminated samples (Samples "A") was subjected to any embossing or aperturing nor were they laminated or otherwise joined to one another.

Samples designated "B"were subjected to a lamination process such as is shown in Figure 3 of the drawings using an embossing and aperturing design such as is shown in Figure 2 of the drawings. The embossing pins had an embossing depth of 0.8 mm and the aperturing pins had an aperturing depth of 0.4 mm. The 24 gsm material corresponded to the first layer 20 of the laminate 10 and the 30 gsm material corresponded to the second layer 40 of the laminate 10.

Samples A and B were formed and tested as: two layers of nonwoven (non-laminated) versus a two layer laminate as disclosed herein; four layers of nonwoven (non-laminated) versus two, two layer laminates as disclosed herein; and, six layers of nonwoven (non-laminated) versus three, two layer laminates as disclosed herein. In the four and six layer versions, the layers were alternated (24 gsm/30 gsm/24 gsm/30 gsm for the four layer version and 24 gsm/30 gsm/24 gsm/30 gsm/24 gsm/30 gsm for the six layer version).

Each of the non-laminated and laminated samples measured 300 mm by 100 mm. For each sample, the thickness was measured under a load of 700 grams. Each sample was placed on a standard laboratory table at room temperature with the 24 gsm layer being the uppermost layer of the respective non-laminated (A) and laminated (B) samples. The laminated samples were stacked with the first layer top surfaces 21 of each laminate 10 facing upwardly, away from the surface of the laboratory table.

A 700 gram weight measuring 120 mm long by 80 mm wide by 26 thick was used to compress each of the above-described samples. The side measuring 120 mm by 80 mm was centered on and contacted each of the samples and left on the samples for a period of three hours. At the end of the three hour period, the 700 gram weight was removed and the samples were left on the table undisturbed for an additional 30 minutes before a final thickness measurement was taken. All thicknesses (original, end of three hours and at three and one-half hours) were measured using the thickness test described above using a 120 gram load. The results of these measurements are set forth in Table I below. "Thickness 1" was the thickness of the layers or laminate(s) as the case may be before the 700 gram load was applied. "Thickness 2" was the thickness of the layers or laminate(s) after the above load had been removed at the end of the three hour period. "Thickness 3" was the thickness after the 30 minute resting period had ended. All three thickness measurements were done according to the above thickness testing procedure under a load of 120 grams. Each value reported in Table 1 is for an average of three samples. The "Tolerance" for each Thickness (Thickness 1, Thickness 2 and Thickness 3) is the thickness value for Sample A minus the thickness of Sample B. The "percent recovery" for each of the Samples A and B was the value of Thickness 3 divided by the Thickness 1 with the quotient multiplied by 100. The "Difference" was the percent recovery of Sample B divided by the percent recovery of Sample A with the quotient multiplied by 100.

**Table I**

| | | 2 Layers of NW/1 Layers of embossed and apertured laminate | | | 4 Layers of NW/2 Layers of embossed and apertured laminate | | | 6 Layers of NW/3 Layers of embossed and apertured laminate | | |
|---|---|---|---|---|---|---|---|---|---|---|
| | | Thickness 1 | Thickness 2 | Thickness 3 | Thickness 1 | Thickness 2 | Thickness 3 | Thickness 1 | Thickness 2 | Thickness 3 |
| A | Non-layered thickness without bonding /mm | 1.08 | 0.90 | 1.03 | 2.12 | 1.98 | 2.13 | 3.03 | 2.65 | 3.01 |
| B | Embossed and apertured laminate thickness/mm | 1.65 | 1.37 | 1.55 | 3.13 | 2.71 | 2.82 | 5.98 | 5.08 | 5.69 |
| | Tolerance | 0.57 | 0.47 | 0.52 | 1.01 | 0.73 | 0.69 | 2.95 | 2.43 | 2.68 |

| | % Recovery | 2 Layers of NW/1 Layer of embossed and apertured laminate | | | 4 Layers of NW/2 Layers of embossed and apertured laminate | | | 6 Layers of NW/3 Layers of embossed and apertured laminate | | |
|---|---|---|---|---|---|---|---|---|---|---|
| | A | 95.3 | | | 100+ | | | 99.3 | | |
| | B | 93.9 | | | 90.1 | | | 95.2 | | |
| | Difference | | | | | | | | | |
| | B/Ax100 | 98.5 | | | 90.1 | | | 95.8 | | |

As can be seen from Table I above, in all cases the non-laminated samples (Samples A) had a higher percentage recovery of their thickness after the load was removed [(Thickness 3 ÷ Thickness 1) x 100] versus the laminate materials of the present invention. However, the laminates while not recovering to the level of the non-laminates, did have differences in recovery [(Sample B recovery ÷ Sample A recovery) x 100] that were very close to the non-laminates (98.5%; 90.1%; and 95.8%). What this demonstrates is that the laminates 10 with the air gaps 35, were able to maintain a high level of their separation between the first layer 20 and the second layer 40 due to the first layer embossments 23. As a result, even in use, such as when the laminate 10 is used as a topsheet 210 in the absorbent articles 200 and 202 shown in Figures 6 and 7, the laminate 10 is able to maintain the air gap 35 which is important in maintaining separation, loft, fluid intake capability and reduced rewet when employed in such products.

## Claims

1. A process for forming a laminate (10) including a fibrous nonwoven web first layer and a second layer comprising:
providing a first roll (110) having a first roll surface and a second roll (120) having a second roll surface; said first roll having a first plurality of embossing pins (112) extending outwardly from the first roll surface for forming a first plurality of embossments (23); at least one of said first roll and said second roll having a first plurality of aperturing pins (114) extending outwardly from the respective first roll surface or second roll surface for forming a first plurality of apertures (24);
positioning said first roll surface of said first roll (110) and said second roll surface of said second roll (120) in mating relationship to form a nip (122) between said first roll surface of said first roll and said second roll surface of said second roll with said first plurality of embossing pins (112) and said first plurality of aperturing pins (114) defining a first roll embossing region (111) in said nip between said first roll and said second roll, said first roll further defining a first roll merging region (113), a first roll bonding region (115) and a first roll take-off region (117);
providing a bonding apparatus (140) adjacent said first roll surface of said first roll in said first roll bonding region (115);
rotating each of said first roll (110) and said second roll (120) in a counter-rotating relationship with respect to one another;
providing a first layer (20) formed of a fibrous nonwoven web; said first layer having a first layer top surface (21) and a first layer bottom surface (22) separated by a first layer thickness (25);
providing a second layer (40) having a second layer upper surface (41) and a second layer lower surface (42) separated by a second layer thickness (45);
feeding said first layer top surface (21) of said first layer into said nip (122) adjacent said first roll surface of said first roll to simultaneously form a first plurality of embossments and a first plurality of apertures in said first layer in said first roll embossing region (111) of said first roll;
feeding said second layer upper surface (41) of said second layer onto said first layer bottom surface (22) of said first layer in said first roll merging region (113) of said first roll; said second layer upper surface (41) of said second layer contacting said first plurality of embossments (23) in said first layer;
bonding with said bonding apparatus (140) said second layer upper surface (41) of said second layer to said first plurality of embossments (23) of said first layer in said first roll bonding region of said first roll to form said laminate (10) with an air gap (35) located between said first layer bottom surface (22) of said first layer and said second layer upper surface (41) of said second layer; and removing said laminate (10) from said first roll in said first roll take-off region (117) of said first roll.

2. The process of claim 1 wherein said second roll (120) has a first plurality of embossing recesses (123) located in the second roll surface with said first plurality of embossing pins mating with said first plurality of embossing recesses.

3. The process of claim 2 wherein the other one of said first roll (110) and said second roll (120) has a first plurality of aperturing recesses (124) located in the respective first roll surface or second roll surface with said first plurality of aperturing pins mating with said first plurality of aperturing recesses.

4. The process of claim 1, which further comprises the step of aperturing said second layer (40) prior to feeding said second layer into said first roll merging region (113) of said first roll.

5. The process of claims 1 wherein said bonding step causes apertures to be formed in an area where said second layer upper surface of said second layer is bonded to at least a portion of said first plurality of embossments in said first layer.

6. A laminate (10) comprising a first fibrous nonwoven layer (20) and a second layer (40),
said first layer (20) having a first layer top surface (21) and a first layer bottom surface (22) defining a first layer thickness (25) therebetween, said first layer defining a plurality of first layer apertures (24) therein at least a portion of which extend from said first layer top surface to said first layer bottom surface to create passageways therebetween,
said second layer (40) comprising a second layer upper surface (41) and a second layer lower surface (42) defining a second layer thickness (45) therebetween,
said first layer bottom surface (22) of said first layer being in spaced apart relationship to said second layer (41) upper surface of said second layer,
**characterised by** said first layer (20) comprising a plurality of first layer embossments (23), at least a portion of which begin in said first layer top surface and define embossment openings, said first layer embossments (23) depending downwardly with embossment side walls (29) and embossment bottoms (30), said embossment bottoms located in said first layer bottom surface of said first layer, and
said first fibrous layer and said second layer being joined to each other at a plurality of embossment bond points (31), at least a portion of said embossment bottoms of said first layer embossments being bonded to said second layer upper surface of said second layer by way of said embossment bond points whereby an air gap (35) is formed between said first layer bottom surface of said first layer and said second layer upper surface of said second layer in an area between said embossment bond points.

7. The laminate of claim 6 wherein said second layer (40) defines a plurality of spaced-apart second layer apertures (47) therein at least a portion of which extend from said second layer upper surface of said second layer to said second layer lower surface of said second layer to create liquid passageways therebetween.

8. The laminate of claim 6 wherein said at least a portion of said plurality of first layer embossment side walls (29) define apertures therein.

9. The laminate of claim 6 wherein said second layer (40) is a fibrous nonwoven web or an apertured film.

10. An absorbent article comprising a liquid permeable top sheet (210) and a liquid impermeable outer cover with an absorbent core positioned therebetween, said top sheet (210) comprising the laminate (10) of claim 6.

11. The absorbent article of claim 10 wherein said article further comprises a liquid acquisition layer positioned between said topsheet (210) and said absorbent core, wherein said top sheet is optionally bonded to said liquid acquisition layer.

12. The absorbent article of claim 10 wherein said article is a diaper, or feminine hygiene product.

## Patentansprüche

1. Verfahren zum Bilden eines Laminats (10), das eine erste Schicht aus einer faserigen Vliesbahn und eine zweite Schicht beinhaltet, umfassend:
Bereitstellen einer ersten Walze (110), die eine erste Walzenfläche aufweist, und einer zweiten Walze (120), die eine zweite Walzenfläche aufweist, wobei die erste Walze eine erste Vielzahl von Prägestiften (112) aufweist, die sich von der ersten Walzenfläche nach außen erstrecken, um eine erste Vielzahl von Prägungen (23) zu bilden; wobei zumindest entweder die erste Walze oder die zweite Walze eine erste Vielzahl von Öffnungsstiften (114) aufweist, die sich von der jeweiligen ersten Walzenfläche oder zweiten Walzenfläche nach außen erstrecken, um eine erste Vielzahl von Öffnungen (24) zu bilden;
Positionieren der ersten Walzenfläche der ersten Walze (110) und der zweiten Walzenfläche der zweiten Walze (120) in Passbeziehung, um einen Spalt (122) zwischen der ersten Walzenfläche der ersten Walze und der zweiten Walzenfläche der zweiten Walze zu bilden, wobei die erste Vielzahl von Prägestiften (112) und die erste Vielzahl von Öffnungsstiften (114) einen ersten Walzenprägebereich (111) in dem Spalt zwischen der ersten Walze und der zweiten Walze definieren, wobei die erste Walze ferner einen ersten Walzenzusammenführungsbereich (113), einen ersten Walzenbondbereich (115) und einen ersten Walzenabnahmebereich (117) definiert;
Bereitstellen einer Bondvorrichtung (140), die an die erste Walzenfläche der ersten Walze in dem ersten Walzenbondbereich (115) angrenzt;
Drehen sowohl der ersten Walze (110) als auch der zweiten Walze (120) in gegenläufiger Beziehung zueinander;
Bereitstellen einer ersten Schicht (20), die aus einer faserigen Vliesbahn gebildet ist, wobei die erste Schicht eine erste Schichtoberfläche (21) und eine erste Schichtunterfläche (22) aufweist, die durch eine erste Schichtdicke (25) getrennt sind;
Bereitstellen einer zweiten Schicht (40), die eine zweiten Schichtoberfläche (41) und eine zweite Schichtunterfläche (42) aufweist, die durch eine zweite Schichtdicke (45) getrennt sind;
Zuführen der ersten Schichtoberfläche (21) der ersten Schicht in den Spalt (122), der an die erste Walzenfläche der ersten Walze angrenzt, um gleichzeitig eine erste Vielzahl von Prägungen und eine erste Vielzahl von Öffnungen in der ersten Schicht in dem ersten Walzenprägebereich (111) der ersten Walze zu bilden;
Zuführen der zweiten Schichtoberfläche (41) der zweiten Schicht auf die erste Schichtunterfläche (22) der ersten Schicht in dem ersten Walzenzusammenführungsbereich (113) der ersten Walze, wobei die zweite Schichtoberfläche (41) der zweiten Schicht die erste Vielzahl von Prägungen (23) in der ersten Schicht kontaktiert;
Bonden, mit der Bondvorrichtung (140), der zweiten Schichtoberfläche (41) der zweiten Schicht an die erste Vielzahl von Prägungen (23) der ersten Schicht in dem ersten Walzenbondbereich der ersten Walze, um das Laminat (10) mit einem Luftspalt (35) zu bilden, der sich zwischen der ersten Schichtunterfläche (22) der ersten Schicht und der zweiten Schichtoberfläche (41) der zweiten Schicht befindet; und Entfernen des Laminats (10) von der ersten Walze in dem ersten Walzenabnahmebereich (117) der ersten Walze.

2. Verfahren nach Anspruch 1, wobei die zweite Walze (120) eine erste Vielzahl von Prägevertiefungen (123) aufweist, die in der zweiten Walzenfläche angeordnet sind, wobei die ersten Vielzahl von Prägestiften mit der ersten Vielzahl von Prägevertiefungen zusammenpassen.

3. Verfahren nach Anspruch 2, wobei die andere der entweder ersten Walze (110) oder der zweiten Walze (120) eine erste Vielzahl von Öffnungsvertiefungen (124) aufweist, die sich in der jeweiligen ersten Walzenfläche oder der zweiten Walzenfläche befinden, wobei die erste Vielzahl von Öffnungsstiften mit der ersten Vielzahl von Öffnungsvertiefungen zusammenpasst.

4. Verfahren nach Anspruch 1, das ferner den Schritt des Öffnens der zweiten Schicht (40) vor dem Zuführen der zweiten Schicht in den ersten Walzenzusammenführungsbereich (113) der ersten Walze umfasst.

5. Verfahren nach Anspruch 1, wobei der Schritt des Bondens bewirkt, dass Öffnungen in einem Bereich gebildet werden, in dem die zweite Schichtoberfläche der zweiten Schicht an zumindest einem Teil der ersten Vielzahl von Prägungen in der ersten Schicht gebondet wird.

6. Laminat (10), das eine erste faserige Vliesschicht (20) und eine zweite Schicht (40) umfasst,
wobei die erste Schicht (20) eine erste Schichtoberfläche (21) und eine erste Schichtunterfläche (22) aufweist, die eine erste Schichtdicke (25) dazwischen definieren, wobei die erste Schicht eine Vielzahl von ersten Schicht-Öffnungen (24) darin definiert, wobei zumindest ein Teil davon sich von der ersten Schichtoberfläche zu der ersten Schichtunterfläche erstreckt, um Durchgänge dazwischen zu erzeugen,
wobei die zweite Schicht (40) eine zweite Schichtoberfläche (41) und eine zweite Schichtunterfläche (42) umfasst, die eine zweite Schichtdicke (45) dazwischen definieren,
wobei die erste Schichtunterfläche (22) in einer beabstandeten Beziehung zu der zweiten Schichtoberfläche (41) der zweiten Schicht steht,
**dadurch gekennzeichnet, dass** die erste Schicht (20) eine Vielzahl von ersten Schicht-Prägungen (23) umfasst, von denen zumindest ein Teil in der ersten Schichtoberfläche beginnt und Prägeöffnungen definieren, wobei die ersten Schicht-Prägungen (23) mit Präge-Seitenwänden (29) und Präge-Böden (30) nach unten abhängig sind, wobei sich die Präge-Böden in der ersten Schichtunterfläche der ersten Schicht befinden; und
wobei die erste faserige Schicht und die zweite Schicht an einer Vielzahl von Prägebondpunkten (31) miteinander verbunden sind, wobei zumindest ein Teil der Präge-Böden der ersten Schicht-Prägungen mit der zweiten Schichtoberfläche der zweiten Schicht über die Prägebondpunkte verbunden ist, wobei ein Luftspalt (35) zwischen der ersten Schichtunterfläche der ersten Schicht und der zweiten Schichtoberfläche der zweiten Schicht in einem Bereich zwischen den Prägebondpunkten gebildet wird.

7. Laminat nach Anspruch 6, wobei die zweite Schicht (40) eine Vielzahl von voneinander beabstandeten zweiten Schicht-Öffnungen (47) definiert, wobei sich zumindest ein Teil davon von der zweiten Schichtoberfläche der zweiten Schicht zu der zweiten Schichtunterfläche der zweiten Schicht erstreckt, um Flüssigkeitsdurchgänge dazwischen zu erzeugen.

8. Laminat nach Anspruch 6, wobei zumindest ein Teil der Vielzahl von Präge-Seitenwänden der ersten Schicht (29) Öffnungen darin definiert.

9. Laminat nach Anspruch 6, wobei die zweite Schicht (40) eine faserige Vliesbahn oder eine mit Öffnungen versehene Folie ist.

10. Absorbierender Artikel, umfassend eine flüssigkeitsdurchlässige Deckschicht (210) und eine flüssigkeitsundurchlässige äußere Abdeckung mit einem dazwischen angeordneten absorbierenden Kern, wobei die Deckschicht (210) das Laminat (10) nach Anspruch 6 umfasst.

11. Absorbierender Artikel nach Anspruch 10, wobei der Artikel ferner eine Flüssigkeitserfassungsschicht umfasst, die zwischen der Deckschicht (210) und dem absorbierenden Kern angeordnet ist, wobei die Deckschicht optional mit der Flüssigkeitserfassungsschicht verbunden ist.

12. Absorbierender Artikel nach Anspruch 10, wobei der Artikel eine Windel oder ein weibliches Hygieneprodukt ist.

## Revendications

1. Procédé de formation d'un stratifié (10) comprenant une première couche de nappe non tissée fibreuse et une deuxième couche comprenant :
la fourniture d'un premier rouleau (110) ayant une première surface de rouleau et d'un deuxième rouleau (120) ayant une deuxième surface de rouleau ; ledit premier rouleau ayant une première pluralité de broches d'embossage (112) s'étendant vers l'extérieur à partir de la première surface de rouleau pour la formation d'une première pluralité d'embossages (23) ; au moins un dudit premier rouleau et dudit deuxième rouleau ayant une première pluralité de broches d'ouverture (114) s'étendant vers l'extérieur à partir de la première surface de rouleau respective ou de la deuxième surface de rouleau pour la formation d'une première pluralité d'ouvertures (24) ;
le positionnement de ladite première surface de rouleau dudit premier rouleau (110) et de ladite deuxième surface de rouleau dudit deuxième rouleau (120) en relation de couplage pour former un pincement (122) entre ladite première surface de rouleau dudit premier rouleau et ladite deuxième surface de rouleau dudit deuxième rouleau avec ladite première pluralité de broches d'embossage (112) et ladite première pluralité de broches d'ouverture (114) définissant une première région d'embossage de rouleau (111) dans ledit pincement entre ledit premier rouleau et ledit deuxième rouleau, ledit premier rouleau définissant en outre une première région de fusionnement de rouleaux (113), une première région de collage de rouleaux (115) et une première région de tirage de rouleaux (117) ;
la fourniture d'un dispositif de collage (140) à côté de ladite première surface de rouleau dudit premier rouleau dans ladite première région de collage de rouleau (115) ;
la rotation de chacun dudit premier rouleau (110) et dudit deuxième rouleau (120) dans une relation de contre-rotation l'un par rapport à l'autre ;
la fourniture d'une première couche (20) formée d'une nappe non tissée fibreuse ; ladite première couche ayant une surface supérieure de première couche (21) et une surface inférieure de première couche (22) séparées par une première épaisseur de couche (25) ;
la fourniture d'une deuxième couche (40) ayant une surface supérieure de deuxième couche (41) et une surface inférieure de deuxième couche (42) séparées par une deuxième épaisseur de couche (45) ;
l'alimentation de ladite surface supérieure de première couche (21) de ladite première couche dans ledit pincement (122) à côté de ladite première surface de rouleau dudit premier rouleau pour former simultanément une première pluralité d'embossages et une première pluralité d'ouvertures dans ladite première couche dans ladite première région d'embossage de rouleau (111) dudit premier rouleau ;
l'introduction de ladite surface supérieure de deuxième couche (41) de ladite deuxième couche sur ladite surface inférieure de première couche (22) de ladite première couche dans ladite première région de fusionnement de rouleau (113) dudit premier rouleau ; ladite surface supérieure de deuxième couche (41) de ladite deuxième couche entrant en contact avec ladite première pluralité d'embossages (23) dans ladite première couche ;
le collage avec ledit dispositif de liaison (140) de ladite surface supérieure de deuxième couche (41) de ladite deuxième couche avec la ladite première pluralité d'embossages (23) de ladite première couche dans ladite première région de collage de rouleau dudit premier rouleau pour former ledit stratifié (10) avec un espace d'air (35) situé entre ladite surface inférieure de première couche (22) de ladite première couche et ladite surface supérieure de deuxième couche (41) de ladite deuxième couche ; et l'extraction dudit stratifié (10) à partir dudit premier rouleau dans ladite première région de prélèvement de rouleau (117) dudit premier rouleau.

2. Procédé selon la revendication 1, dans lequel ledit deuxième rouleau (120) a une première pluralité de récessions d'embossage (123) situées dans la deuxième surface de rouleau avec ladite première pluralité de broches d'embossage en couplage avec ladite première pluralité de récessions d'embossage.

3. Procédé selon la revendication 2, dans lequel l'autre dudit premier rouleau (110) et dudit deuxième rouleau (120) a une première pluralité de récessions d'ouvertures (124) située dans la première surface de rouleau respective ou la deuxième surface de rouleau avec ladite première pluralité de broches d'ouverture en couplage avec ladite première pluralité de récessions d'ouvertures.

4. Procédé selon la revendication 1, qui comprend en outre l'étape d'ouverture de ladite deuxième couche (40) avant l'introduction de ladite deuxième couche dans ladite première région de fusionnement de rouleau (113) dudit premier rouleau.

5. Procédé selon les revendications 1, dans lequel ladite étape de collage provoque la formation d'ouvertures dans une zone où ladite surface supérieure de deuxième couche de ladite deuxième couche est collée à au moins une partie de ladite première pluralité d'embossages dans ladite première couche.

6. Un stratifié (10) comprenant une première couche non tissée fibreuse (20) et une deuxième couche (40),
ladite première couche (20) ayant une surface supérieure de première couche (21) et une surface inférieure de première couche (22) définissant une première épaisseur de couche (25) entre elles, ladite première couche définissant une pluralité d'ouvertures de première couche (24) à l'intérieur dont au moins une partie s'étend de ladite surface supérieure de première couche à ladite surface inférieure de première couche pour créer des passages entre elles,
ladite deuxième couche (40) comprenant une surface supérieure de deuxième couche (41) et une surface inférieure de deuxième couche (42) définissant une deuxième épaisseur de couche (45) entre elles,
ladite surface inférieure de première couche (22) de ladite première couche étant en relation espacée avec ladite surface supérieure de deuxième couche (41) de ladite deuxième couche,
**caractérisé par** ladite première couche (20) comprenant une pluralité d'embossages de première couche (23), dont au moins une partie commence dans ladite surface supérieure de première couche et définit des ouvertures d'embossage, lesdits embossages de première couche (23), dépendant vers le bas avec les parois latérales d'embossage (29) et des fonds d'embossage (30), lesdits fonds d'embossage étant situés dans ladite surface inférieure de première couche de ladite première couche, et
ladite première couche fibreuse et ladite deuxième couche étant reliées l'une à l'autre sur une pluralité de points de collage d'embossage (31), au moins une partie desdits fonds d'embossage desdits embossages de première couche étant collée à ladite surface supérieure de deuxième couche de ladite deuxième couche par le biais desdits points de collage d'embossage par lesquels un écart d'air (35) est formé entre ladite surface inférieure de première couche de ladite première couche et ladite surface supérieure de deuxième couche de ladite deuxième couche dans une zone entre lesdits points de collage d'embossage.

7. Stratifié selon la revendication 6, dans lequel ladite deuxième couche (40) définit une pluralité d'ouvertures de deuxième couche espacées (47) dans au moins une partie s'étend à partir de ladite surface supérieure de deuxième couche de ladite deuxième couche à ladite surface inférieure de deuxième couche de ladite deuxième couche pour créer des passages liquides entre elles.

8. Stratifié selon la revendication 6, dans lequel ladite au moins une partie de ladite pluralité de parois latérales d'embossage de première couche (29) y définit des ouvertures.

9. Stratifié selon la revendication 6, dans lequel ladite deuxième couche (40) est une nappe non tissée fibreuse ou un film à ouvertures.

10. Article absorbant comprenant une feuille supérieure perméable aux liquides (210) et un revêtement extérieur imperméable aux liquides avec un centre absorbant positionné entre eux, ladite feuille supérieure (210) comprenant le stratifié (10) selon la revendication 6.

11. Article absorbant selon la revendication 10, dans lequel ledit article comprend en outre une couche d'acquisition de liquide positionnée entre ladite feuille supérieure (210) et ledit centre absorbant, dans lequel ladite feuille supérieure est facultativement collée à ladite couche d'acquisition de liquide.

12. Article absorbant selon la revendication 10, dans lequel ledit article est une couche ou un produit d'hygiène féminine.
